# EUROPEAN PATENT APPLICATION

(11) **EP 4 201 439 A1**
(43) Date of publication of application: **28.06.2023**
(21) Application number: 21217588.9
(22) Date of filing: 23.12.2021
(51) Int. Cl.: A61L 27/36, A61L 27/38, A61L 27/40, A61L 27/44, A61L 27/58, A61L 27/60, A61K 9/00

(54) **A THREE-DIMENSIONAL BIOCOMPATIBLE MATRIX AND ITS USES IN WOUND MANAGEMENT**

(71) Applicant: Axenoll Life Sciences AG, 8001 Zurich (CH)
(72) Inventor: DECK, David L., 98000 Monaco (MC); PLÖGER, Frank, 69245 Bammental (DE); ELSTER, Dana, 07745 Jena (DE); SCHWARZ, Nicolle, 07745 Jena (DE); TADIC KRIPPENDORF, Vedrana, 07745 Jena (DE)
(74) Representative: Engelhard, Markus

(57) **Abstract**

The present invention relates to a three-dimensional biocompatible matrix comprising a three-dimensional scaffold made of a polymeric agent, and comprising at least one further component. The present invention also relates to a composition comprising such three-dimensional biocompatible matrix. Furthermore, the present invention also relates to uses of such three-dimensional biocompatible matrix and/or composition. In particular, the present invention also relates to such three-dimensional biocompatible matrix and/or composition for use in methods of wound treatment or management. Moreover, the present invention also relates to a method for producing a three-dimensional biocompatible matrix according to the present invention. Furthermore, the present invention relates to an ink composition for three-dimensional (₃D) printing and its uses in producing a three-dimensional biocompatible matrix according to the present invention.

## Description

### FIELD OF THE INVENTION

The present invention relates to a three-dimensional biocompatible matrix comprising a three-dimensional scaffold made of a polymeric agent, and comprising at least one further component. The present invention also relates to a composition comprising such three-dimensional biocompatible matrix. Furthermore, the present invention also relates to uses of such three-dimensional biocompatible matrix and/or composition. In particular, the present invention also relates to such three-dimensional biocompatible matrix and/or composition for use in methods of wound treatment or management. Moreover, the present invention also relates to a method for producing a three-dimensional biocompatible matrix according to the present invention. Furthermore, the present invention relates to an ink composition for three-dimensional (3D) printing and its uses in producing a three-dimensional biocompatible matrix according to the present invention.

### BACKGROUND OF THE INVENTION

Due to the complexity of the processes involved in the healing process, it can take considerable periods of time until a wound heals and becomes closed. Frequently, wounds are painful and their healing can be aggravated by infections which may lead to further complications. The medical treatment of a wound, also referred to as "wound management" ideally should support healing of a wound, prevent wound infections and alleviate pain caused by such wound. Moreover the formation of scars should be reduced or prevented altogether. An important aspect of wound management is that the natural process of such healing should be promoted by any medical activity, whilst not being negatively affected by it. The management of wounds caused by burns has received particular attention due to the frequent occurrence of such burn wounds. They may be caused by fire, radiation, exposure to electrical currents, to chemicals or to friction. Depending on the severity of the burn, burn injuries/wounds are classified into different categories:
In first-degree burns only the epidermis is affected, and there are only minor damages to the skin. Healing of such injuries typically occurs spontaneously and without the formation of scars. A typical example of such first-degree burn is a mild sunburn. A conservative treatment with skin care creams is recommended but not strictly necessary. An antimicrobial treatment or a wound dressing are normally not required in such types of burns.

Second-degree burns which are also sometimes referred to as "partial thickness burns" affect both the epidermis and part of the dermis. The location of the burn looks red, blistered and may be swollen and painful. The injury may ooze or bleed. Such types of burns usually heal within 1 to 3 weeks; however, after healing, the skin may be discoloured. These burns generally do not leave raised scars, and treatment may vary, depending on the depth of such burn. It may include ointments or special dressings. Surgery may be necessary for very deep second-degree burns. In some instances, part of the treatment involves the removal of necrotic tissue as well as antimicrobial treatment.

Third-degree burns destroy the epidermis and dermis and may extend into the innermost layer of skin, i.e. subcutaneous tissue. The location of the burn may look white or blackened and charred; in some instances, sensation in the skin may be affected because of the destruction of nerve endings. Treatment involves the removal of necrotic tissue and antimicrobial treatment due to the considerable risk of bacterial infections. Treatment may also involve skin grafts or temporary coverage by skin substitutes.

Fourth-degree burns extend through both layers of the skin, subcutaneous tissue as well as deeper lying tissues, possibly involving muscle and bone. Treatment involves surgery, skin grafts and, in many instances amputation of affected extremities.

Generally speaking, there are various options for the treatment of burn injuries, and depending on the degree of the burn injury, they are used in various combinations. Treatment options involve wound cleaning, removal of necrotic tissue, moisturising, antibiotic wound care using ointments alone or in combination with appropriate dressings and bandages, surgery, skin grafts, and coverage with skin substitutes. Skin grafts maybe used involving the patient's own skin (autologous skin graft), the skin of a healthy person (allogeneic skin graft) or the skin of animals (xenogeneic skin grafts). The use of skin grafts has its limitations particularly in patients where large body areas are affected, due to the limited availability of suitable healthy skin. One way to address this has been to artificially generate skin tissue patches by in vitro cultivation, or to consider the use of artificial skin substitutes emulating the qualities of the natural skin and extracellular matrix of such skin. These methodologies have been used to a variable degree of success.

Accordingly, there is a need in the art to provide for alternatives in the treatment and management of wounds, in particular burn injuries. There is also a need in the art for skin substitutes for wound management that allow for an alleviation of pain and/or a reduction of inflammation of such wound. There is also a need in the art to provide for skin substitutes for wound management that allow a facilitation of wound healing and/or a reconstruction of skin in and/or around the wound. There is also need in the art to provide for skin substitutes that allow a safe and efficient delivery of an analgesic to such wound.

### SUMMARY OF THE INVENTION

In a first aspect, the present invention relates to a three-dimensional biocompatible matrix comprising
- a three-dimensional scaffold made of a polymeric agent, and
- at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm, said at least one component being incorporated in said scaffold.

In one embodiment, said scaffold is dimensionally stable and is not a spreadable semi solid or liquid, such as a cream, lotion, ointment, salve, paste, unguent, or balm.

In one embodiment, said scaffold has been produced by three-dimensional (3D) printing, preferably by three-dimensional (3D) screen printing.

In one embodiment, said scaffold does is not a knitted fabric, not a woven or non-woven, and/or does not have a filamentous structure, wherein, preferably, said scaffold is resorbable.

In another embodiment, said scaffold also does not have a filamentous structure and also is not a knitted fabric, not a woven or non-woven, wherein, however, preferably, in such another embodiment, said scaffold is not resorbable. In such an embodiment, said scaffold is made of a polymer that typically is chemically inert under physiological conditions (as would be encountered in a wound) and that is preferably not prone to degradation or other process leading to its disappearance, e.g. degradation by enzymatic processes. Such a polymer may be a synthetic polymer, such as poly(methyl)methacrylates, polyurethanes, polycaprolactons, polyamides, polydioxanones, and mixtures or combinations of such synthetic polymers, or such as silicone-based polymers, e.g. polysiloxanes, for example polydimethylsiloxanes, or mixtures or combinations thereof.

In one embodiment, said scaffold has a thickness in the range of from 100 µm to 20 mm, preferably from 100 µm to 15 mm, more preferably from 100 µm to 12 mm, even more preferably from 100 µm to 10 mm, wherein even more preferably said scaffold has a minimum thickness in the range of from 100 µm to 200 µm, allowing for the accommodation of a monolayer of cells, in particular of fibroblasts and/or keratinocytes. Typically, when said scaffold contains cells, for example in the form of a contiguous monolayer or patches of such monolayer, this occurs only when the three-dimensional biocompatible matrix has been applied to a wound, and the cells contained within the scaffold originate from the wound tissue or tissue surrounding the wound. This means that in such embodiments prior to application of the three-dimensional biocompatible matrix, such matrix does not contain any cells. It should also be noted that in preferred embodiments, the three-dimensional biocompatible matrix, prior to application to a wound, is sterile and does not contain any microorganism capable of replication and/or growth.

In one embodiment, said three-dimensional biocompatible matrix is in a dry form, i.e. does not comprise a liquid phase. When such three-dimensional biocompatible matrix is in a dry form, preferably, it has been brought into such dry form after manufacturing by an appropriate drying process, such as evaporation or freeze-drying. Such "dry state" embodiment is particularly useful when the three-dimensional biocompatible matrix is to be stored for extended periods of time and/or shipped and/or is not put to any (medical) uses (yet). In another embodiment, said three-dimensional biocompatible matrix further comprises a liquid phase, such that the three-dimensional scaffold is in a gel-state, preferably a hydrogel-state. Such embodiment is particularly useful and is, in fact, a preferred embodiment when the three-dimensional biocompatible matrix is about to be used for medical purposes, e.g. in methods of treating and/or managing wounds. When the three-dimensional scaffold is in a hydrogel-state, the liquid phase is water or an aqueous solution. Also, when the three-dimensional scaffold is in a hydrogel state, and the three-dimensional biocompatible matrix is nevertheless to be stored for some time, it is preferred that the three-dimensional biocompatible matrix is enclosed in a sealed compartment, such as a sealed bag or pouch, such that the liquid phase may not evaporate.

In one embodiment, the three-dimensional biocompatible matrix comprises
a)
   - a scaffold made of a polymeric agent, and
   - a non-opioid analgesic, incorporated in said scaffold or
b)
   - a scaffold made of a polymeric agent, and
   - extracellular vesicles derived from animal cells, preferably derived from mammalian cells, more preferably derived from human cells, incorporated in said scaffold; or
c)
   - a scaffold made of a polymeric agent, and
   - artificially constructed lipid vesicles having a size in the range of from 20 nm to 150 nm, incorporated in said scaffold; or
d)
   - a scaffold made of a polymeric agent, and
   - a non-opioid analgesic and extracellular vesicles derived from animal cells, preferably derived from mammalian cells, more preferably derived from human cells, both said non-opioid analgesic and said extracellular vesicles being incorporated in said scaffold; or
e)
   - a scaffold made of a polymeric agent, and
   - a non-opioid analgesic and artificially constructed lipid vesicles having a size in the range of from 20 nm to 150 nm, both said non-opioid analgesic and said artificially constructed lipid vesicles being incorporated in said scaffold; or
f)
   - a scaffold made of a polymeric agent, and
   - non-opioid analgesic and extracellular vesicles derived from animal cells, preferably derived from mammalian cells, more preferably derived from human cells and artificially constructed lipid vesicles having a size in the range of from 20 nm to 150 nm, each of them being incorporated in said scaffold.

In one embodiment, said polymeric agent is selected from the group comprising a) naturally occurring polymers selected from polysaccharides, e.g. agarose, chitin, chitosan, dextran, alginate, carrageenan, cellulose, starch, fucoidan, laminaran, glycosaminoglycans, copolymers of glycosaminoglycan and collagen; gums selected from xanthan gum, arabic gum, ghatti gum, guar gum, locust bean gum, tragacanth gum, karaya gum; and inulin; polypeptides, e.g. collagen, and hydrolysed forms of such polypeptides, e.g. gelatin; poly-amino acids, such as poly-lysine; polynucleotides; b) synthetic polymers, such as poly(alpha-hydroxy acids), such as poly lactic acid (PLA), poly glycolic acid (PGA), poly lactic-co-glycolic acid (PLGA), poly(alkyl)methacrylates, e.g. poly(methyl)methacrylates, polyvinylpyrrolidone (PVP), polyethylene glycol (PEG), polyurethanes, polycaprolactons, polyamides, polydioxanones, polyglycerol, and mixtures or combinations of such synthetic polymers b) with such naturally occurring polymers a); c) silicone-based polymers, such as polysiloxanes, e.g. polydimethylsiloxanes; and combinations or mixtures of any of the foregoing of a) - c).

Preferred embodiments of glycosaminoglycans are hyaluronic acid, heparin, heparan sulphate, chondroitin sulphate, and keratane sulphate.

As used herein, the term "cellulose" is meant to include also cellulose derivatives such as cellulose ether derivatives, e.g. methyl cellulose, ethyl cellulose, propyl cellulose hydroxypropyl cellulose, hydroxypropylmethyl cellulose, hydroxyethyl cellulose, carboxymethyl cellulose; and cellulose ester derivatives, such as cellulose acetate (CA), cellulose acetate phthalate (CAP), cellulose acetate butyrate (CAB), Cellulose acetate trimelitate (CAT), and hydroxypropylmethyl cellulose phthalate (HPMCP).

In one embodiment, said polymeric agent is selected from naturally occurring polymers selected from polysaccharides, e.g. agarose, chitin, chitosan, dextran, alginate, carrageenan, cellulose, starch fucoidan, laminaran, glycosaminoglycans, copolymers of glycosaminoglycan and collagen; gums selected from xanthan gum, arabic gum, ghatti gum, guar gum, locust bean gum, tragacanth gum, karaya gum; and inulin; polypeptides, e.g. collagen, and hydrolysed forms of such polypeptides, e.g. gelatin; poly-amino acids, such as poly-lysine; polynucleotides; wherein, preferably said polymeric agent is selected from alginate, gelatin, cellulose, collagen, chitosan, and mixtures of any of the foregoing..

In one embodiment, said non-opioid analgesic is selected from amino ester analgesics, amino amide analgesics, fomocains and respective carbonic acid adducts of said amino ester analgesics or of said amino amide analgesics or of said fomocains, wherein preferably, said amino ester analgesics include procaine, chloroprocaine, oxybuprocaine, benzocaine, tetracaine, and proxymetacaine; and wherein, preferably, said amino amide analgesics include lidocaine, mepivacaine, prilocaine, articaine, bupivacaine, ropivacaine, cinchocaine, and etidocaine; and wherein, preferably, said fomocains include fomocaine and C-alkylmorpholine derivatives thereof.

In one embodiment, said non-opioid analgesic is an amino ester analgesic or a carbonic acid adduct thereof, preferably selected from procaine, chloroprocaine, oxybuprocaine, benzocaine, tetracaine, and proxymetacaine, and their respective carbonic acid adducts.

In one embodiment, said non-opioid analgesic is an amino amide analgesic or a carbonic acid adduct thereof, preferably selected from lidocaine, mepivacaine, prilocaine, articaine, bupivacaine, ropivacaine, cinchocaine, and etidocaine, and their respective carbonic acid adducts.

Carbonic acid adducts of the aforementioned analgesics are known and have for example been described in WO 2019/048590 A1. Particularly preferred analgesics are procaine, chloroprocaine, and lidocaine, and their respective carbonic acid adducts.

In one embodiment, said non-opioid analgesic is selected from acetylsalicylic acid, ibuprofen, diclofenac, naproxen, indomethacin, paracetamol, metamizol, phenazone, propyphenazone, parecoxib, celecoxib, etoricoxib, ketamine, capsaicin, ziconotide, cannabinoids, and flupirtine.

In one embodiment, said extracellular vesicles are derived from animal platelets or stem cells, preferably mammalian platelets or stem cells, more preferably, human platelets or stem cells, and contain at least one component selected from cytokines, growth factors, transcription factors, RNAs, in particular micro RNA, and mRNA; wherein, preferably, said stem cells are selected from mesenchymal stem cells and induced stem cells, wherein, more preferably, said mesenchymal stem cells are derived from bone marrow, umbilical cord blood, adipose tissue, or amniotic fluid; and wherein, preferably, said artificially constructed lipid vesicles contain at least one component selected from cytokines, growth factors, transcription factors, RNAs, in particular micro RNA, mRNA, and a non-opioid analgesic, as defined herein.

In one embodiment, a) said extracellular vesicles are derived from animal platelets, preferably mammalian platelets, more preferably human platelets, and are positive for at least one of cellular markers selected from CD9, CD41a, CD41b, CD42b, CD61, CD62P, CD63 and syntenin, and/or wherein said extracellular vesicles are negative for at least one of cellular markers selected from CD81, CD3, CD4, CD19, CD20, CD2, CD8, CD11a and CD25; or b) said extracellular vesicles are derived from stem cells and are positive for CD81, CD9, CD63, Tsg101, and HSP70, and they are positive for one, several or all of CD105, CD90, CD73 and CD44, and/or wherein said extracellular vesicles are negative for one, several or all of CD45, CD34, CD31, CD19, CD79α, CD14, CD11b, and HLA-DR.

In one embodiment, said three-dimensional biocompatible matrix further comprises cells, in particular fibroblasts and/or keratinocytes, obtained from living tissues or cadaver tissues. If such cells are comprised within the three-dimensional biocompatible matrix, they would be typically already incorporated during the manufacturing of the three-dimensional biocompatible matrix, i.e. they would be comprised by such three-dimensional biocompatible matrix already prior to any application/use thereof.

In another embodiment different to the embodiment of the preceding paragraph, said three-dimensional biocompatible matrix does not further comprise any cells, such as fibroblasts and/or keratinocytes. In particular, in this embodiment, said three-dimensional biocompatible matrix, prior to its application/use does not comprise any cells. This means that during the manufacture of such three-dimensional biocompatible matrix, no cells are included or incorporated in the production thereof, and prior to any application/use of such biocompatible matrix, it is free of any biological cells. Only after its application, for example its placement on a wound, the three-dimensional biocompatible matrix may become colonized by biological cells from the wound or its surroundings.

The present invention also relates to a kit configured for producing a three-dimensional biocompatible matrix, as defined herein, wherein said kit comprises a three-dimensional scaffold made of a polymeric agent, as defined herein, and a container comprising said at least one component as defined herein, wherein preferably, said container comprises a solution or dispersion or suspension of said at least one component in a suitable solvent.

The present invention also relates to a composition comprising a three-dimensional biocompatible matrix according to the present invention, as defined herein.

In one embodiment, the composition according to the present invention is manufactured as a patch, dressing, pad or pouch.

In one embodiment of the composition, said composition has a layered arrangement, for example, a laminate or a multilayer arrangement, in particular where said composition is in use, wherein, preferably, one of the layers of said layered arrangement is formed by said three-dimensional biocompatible matrix. For such embodiment, it is preferred that said composition comprises at least one additional layer that is attached to said three-dimensional biocompatible matrix. Such additional layer that is attached to said three-dimensional biocompatible matrix, may be a carrier layer providing mechanical support for said biocompatible matrix, or is a semipermeable backing layer preventing diffusion of said at least one component from one side of said biocompatible matrix, or is a solvent-impermeable backing layer, in particular a water-impermeable backing layer, sealing said biocompatible matrix on one side and preventing diffusion of said at least one component from said biocompatible matrix on such side. For such embodiment, it is particularly preferred that such layered arrangement has a thickness in the range of from 5 mm to 20 mm, preferably from 5 mm to 15 mm, more preferably from 8 mm to 12 mm, more preferably from 9 mm to 11 mm, and even more preferably approximately 10 mm. In one embodiment, said three-dimensional biocompatible matrix has a thickness in the range of from 100 µm to 20 mm, preferably from 100 µm to 15 mm, more preferably from 100 µm to 12 mm, even more preferably from 100 µm to 10 mm; and wherein said additional layer has a thickness in the range of from 50 µm to 2 mm, preferably from 100 µm to 2 mm, more preferably from 100 µm to 1 mm and even more preferably from 100 µm to 500 µm. The aforementioned embodiment(s) of said composition may also, for example comprise additional components, when said composition is not in use, but is stored or transported; for example it may be attached to a releasable carrier sheet for transport and/or storage of said composition. Such releasable carrier sheet may act as a means for safe transport and/or storage of said composition, but is removed from said composition prior to an intended use of said composition.

In another embodiment of the composition, said composition has a layered arrangement, but only comprises a single layer formed by said three-dimensional biocompatible matrix, in particular where said composition is in use. In such embodiment, when in use, said composition has an arrangement of a single layer, such single layer being made of a polymeric agent, as defined herein, or a combination or mixture of such polymeric agents, as defined herein; such single layer making up the three-dimensional biocompatible matrix. For such embodiment, it is preferred that said composition does not comprise any additional layer or sheet that would be attached to said three-dimensional biocompatible matrix. For such embodiment of a single layer arrangement, it is particularly preferred that such single layer arrangement has a thickness in the range of from 0.5 mm to 3 mm, preferably from 0.5 mm to 2 mm, more preferably in the range of from 1 mm to 2 mm. However, it should be noted that the aforementioned (single layer) embodiment of said composition may also, for example comprise additional components, when said composition is not in use, but is stored or transported; for example it may be attached to a releasable carrier sheet for transport and/or storage of said composition. Such releasable carrier sheet may act as a means for safe transport and/or storage of said composition, but is removed from said composition prior to an intended use of said composition. It should also be noted that when such single layer making up the three-dimensional biocompatible matrix is produced by three-dimensional (3D) screen printing, such screen-printed single layer may be made of several sublayers, due to the inherent modalities of the screen-printing process. However, all of these sublayers are made of the same polymeric agent, as defined herein, or the same combination or mixture of such polymeric agents, as defined herein, such sublayers in their entirety making up the single layer which is the three-dimensional biocompatible matrix.

In one embodiment of the composition, said composition has one of the following two structures A or B:
**structure A:**
   wherein said three-dimensional biocompatible matrix is arranged in a first layer, and wherein said composition comprises an additional layer attached to said first layer, which additional layer is either a carrier layer providing mechanical support for said biocompatible matrix, or is a semipermeable backing layer preventing diffusion of said at least one component from one side of said biocompatible matrix, or is a solvent-impermeable backing layer, in particular a water-impermeable backing layer, sealing said biocompatible matrix on one side and preventing diffusion of said at least one component from said biocompatible matrix on such side, or wherein said additional layer is both a carrier layer and a semipermeable or solvent-impermeable backing layer;
   wherein said first layer has a thickness in the range of from 100 µm to 20 mm, preferably from 100 µm to 15 mm, more preferably from 100 µm to 12 mm, even more preferably from 100 µm to 10 mm; and wherein said additional layer has a thickness in the range of from 50 µm to 2 mm, preferably 100 µm to 2 mm, more preferably from 100 µm to 1 mm, and even more preferably from 100 µm to 500 µm; or
**structure B:**
   wherein said three-dimensional biocompatible matrix is arranged as the only layer within said composition, and there is no additional layer attached to said layer of said three-dimensional biocompatible matrix; and wherein said only layer has a thickness in the range of from 500 µm to 3 mm, preferably in the range of from 500 µm to 2 mm, more preferably in the range of from 1 mm to 2 mm;
   wherein, optionally, said composition of structure A and said composition of structure B may be attached to a releasable carrier sheet for transport and/or storage of said composition, and is not attached to a releasable carrier sheet and does not comprise such releasable carrier sheet, when said composition is in use, e.g. in treating and/or managing a wound.

In one embodiment of said composition having structures A or B, during transport and/or storage, said composition of structures A and B is attached to a releasable carrier sheet for transport and/or storage of said composition, and is not attached to a releasable carrier sheet and does not comprise such releasable carrier sheet, when said composition is in use, e.g. in treating and/or managing a wound.

In a further aspect, the present invention also relates to the three-dimensional biocompatible matrix or the composition according to the present invention, as defined herein, for use in a method of treating and/or managing a wound.

In one embodiment of the three-dimensional biocompatible matrix or the composition for use according to the present invention, said wound is an acute wound or a chronic wound, wherein, preferably, said acute wound is a burn, a skin lesion, a skin injury, a surgical wound, a cut or a stab wound, and wherein, preferably, said chronic wound is a decubitus ulcer or a diabetic ulcer; wherein, more preferably, said burn is selected from thermal burns, radiation burns, chemical burns, burns caused by exposure to electrical current, and friction burns, wherein, even more preferably, said burn is a first degree, second degree or third degree burn, even more preferably a second degree or third degree burn.

In one embodiment of the three-dimensional biocompatible matrix or the composition for use according to the present invention, said method of treating and/or managing a wound comprises administering said three-dimensional biocompatible matrix or said composition to said wound and allowing it to remain in contact with such wound for a defined period of time.

In one embodiment of the three-dimensional biocompatible matrix or the composition for use according to the present invention, said method is for alleviating pain and/or for reducing inflammation of said wound.

In one embodiment of the three-dimensional biocompatible matrix or the composition for use according to the present invention, said method is for increasing the speed at which wound healing is achieved in comparison to a wound that is not treated by said method, and/or wherein said method is for delivery of an analgesic to said wound, and/or wherein said method is for reconstruction of skin in and/or around said wound.

In a further aspect, the present invention also relates to the use of a three-dimensional biocompatible matrix or a composition according to the present invention for the manufacture of a medicament for treating and/or managing a wound. In such aspect, the three-dimensional biocompatible matrix, the composition and the wound are as defined herein. In such aspect, the medicament is to be used in a method of treating and/or managing a wound. More particularly, in such aspect, said method of treating and/or managing a wound comprises administering said three-dimensional biocompatible matrix or said composition to said wound and allowing it to remain in contact with such wound for a defined period of time.

In one embodiment of such aspect, said method in which said medicament is to be used is for alleviating pain and/or for reducing inflammation of said wound.

Moreover, in addition or alternatively to the previous embodiment, said method is for increasing the speed at which wound healing is achieved in comparison to a wound that is not treated by said method, and/or wherein said method is for delivery of an analgesic to said wound, and/or wherein said method is for reconstruction of skin in and/or around said wound.

In a further aspect, the present invention also relates to a method of treating and/or managing a wound, such method comprising administering a three-dimensional biocompatible matrix or a composition according to the present invention, as defined herein, to said wound and allowing it to remain in contact with such wound for a defined period of time. Also in this aspect, in some embodiments, said method is for alleviating pain and/or for reducing inflammation of said wound.

Moreover, in addition or alternatively to the previous embodiment of this aspect, said method is for increasing the speed at which wound healing is achieved in comparison to a wound that is not treated by said method, and/or wherein said method is for delivery of an analgesic to said wound, and/or wherein said method is for reconstruction of skin in and/or around said wound.

In a further aspect, the present invention also relates to a method for producing a three-dimensional biocompatible matrix according to the present invention, as defined herein, said method comprising
Either:
   a) Providing a polymeric agent and 3D-printing, preferably 3D-screen-printing, such agent into a three-dimensional scaffold;
   b) Applying at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm, to said three-dimensional scaffold and allowing said at least one component to be incorporated in said scaffold, thereby producing said three-dimensional biocompatible matrix;
   said polymeric agent and said at least one component being as defined herein;
Or:
   a^{∗}) Providing a polymeric agent and at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm, and mixing said polymeric agent and said at least one component, and thereafter
   b^{∗}) 3D-printing, preferably 3D-screen-printing, the resultant mixture from step a^{∗}) into a three-dimensional scaffold, such that said at least one component is incorporated in said scaffold, thereby producing said three-dimensional biocompatible matrix;
   said polymeric agent and said at least one component being as defined herein.

In one embodiment, said step b) can be performed by any suitable technology, including spraying, soaking, coating, imbibing, impregnating, applying and subsequently freeze-drying, nanosol technology, applying and subsequently exposing scaffold/matrix to negative pressure or vacuum, etc..

In a further aspect, the present invention also relates to an ink composition for three-dimensional (3D) printing, in particular three-dimensional (3D) screen printing, said ink composition comprising:
i. a polymeric agent selected from the group comprising a) naturally occurring polymers selected from polysaccharides, e.g. agarose, chitin, chitosan, dextran, alginate, carrageenan, cellulose, starch fucoidan, laminaran, glycosaminoglycans, copolymers of glycosaminoglycan and collagen; gums selected from xanthan gum, arabic gum, ghatti gum, guar gum, locust bean gum, tragacanth gum, karaya gum; and inulin; polypeptides, e.g. collagen, and hydrolysed forms of such polypeptides, e.g. gelatin; poly-amino acids, such as poly-lysine; polynucleotides; b) synthetic polymers, such as poly(alpha-hydroxy acids), such as poly lactic acid (PLA), poly glycolic acid (PGA), poly lactic-co-glycolic acid (PLGA), poly(alkyl)methacrylates, e.g. poly(methyl)methacrylates, polyvinylpyrrolidone (PVP), polyethylene glycol (PEG), polyurethanes, polycaprolactons, polyamides, and mixtures or combinations of such synthetic polymers b) with such naturally occurring polymers a); c) silicone-based polymers, such as polysiloxanes, e.g. polydimethylsiloxanes; and combinations or mixtures of any of the foregoing of a) - c);
ii. a solvent for i, wherein preferably said solvent is selected from water, aqueous solutions, alcohol, in particular methanol, ethanol, n-propanol, or isopropanol, alcoholic solutions, tetrahydrofuan, acetone, and ethyl acetate;
iii. at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm; wherein said non-opioid analgesic, extracellular vesicles derived from animal cells, preferably derived from mammalian cells, more preferably derived from human cells, and artificially constructed lipid vesicles are as defined herein;
iv. optionally, one or several further components selected from fillers, cross-linkers, binders, surfactants, additives, diluents, thickening agents, colours, dyes, stabilizing agents, buffering agents, humectants, emulsifying agents, dispersing agents, and preserving agents.

In a preferred embodiment, said ink composition for three-dimensional (3D) printing, in particular three-dimensional (3D) screen printing, has a viscosity in the range of from 100 - 300000 mPas, preferably from 1000 to 30000 mPas. Preferably, the values in such viscosity ranges are measured and determined in accordance with the methodology of DIN EN ISO 3219-1 and/or ISO 3219.

In one embodiment of the ink composition, said at least one component is present within said ink composition at a concentration in the range of from 0.1 - 20 wt.%, with reference to the weight of the ink composition. It should be noted that in embodiments, where the at least one component is applied to the three-dimensional (3D) biocompatible matrix, after such matrix has been formed, said at least one component is applied as part of a solution or dispersion or suspension, within which said at least one component is present at a concentration in the range of from 0.1 - 20 wt.%, with reference to the weight of such solution or dispersion or suspension.

### DETAILED DESCRIPTION

The terms "of the [present] invention", "in accordance with the invention", "according to the invention" and the like, as used herein, are intended to refer to all aspects and embodiments of the invention described and/or claim herein. As used herein, the term "comprising" is to be construed as encompassing both "including" and "consisting of', both meanings being specifically and explicitly intended, and hence, individually disclosed embodiments in accordance with the present invention. Where used herein, "and/or" is to be taken as a specific disclosure of each of the two specified features or components with or without the other. For example, "A" and/or "B" is to be taken as a specific disclosure of each of (i) A, (ii) B and (iii) A and B, just as if each is set out individually herein. Where an indefinite or definite article is used, wherein referring to a singular noun, e. g. "a", "an" or "the", this includes a plural of that noun unless something else is specifically stated. Likewise, such disclosure equally is meant to be taken as a specific disclosure of a single individual entity initiated by "a", "an" or "the".

The present inventors have surprisingly found that the inclusion of at least one component selected from a non-opioid analgesic, extracellular vesicles derived from animal cells, preferably derived from mammalian cells, more preferably derived from human cells, and artificially constructed lipid vesicles, into a three-dimensional scaffold made of a polymeric agent results in a three-dimensional biocompatible matrix that can be advantageously used for medical purposes, in particular in methods of treating and/or managing wounds. In preferred embodiments, said three-dimensional scaffold has been produced by three-dimensional (3D) printing, preferably by three-dimensional (3D) screen printing. These 3D printing techniques, in particular 3D screen printing techniques, are very versatile and quite gentle due to the avoidance of harsh conditions, such as high pressure or temperature. As a consequence thereof, the use of 3D printing techniques, in particular of 3D screen printing techniques, in the production of such three-dimensional biocompatible matrix allows for the inclusion and/or incorporation of delicate or sensitive components, such as active pharmaceutical ingredients (APIs) or lipid vesicles or even biological cells, directly during the manufacturing process. As a result of such inclusion and/or incorporation of such delicate or sensitive components directly during the manufacturing process, these components may become integral parts of the three-dimensional scaffold rather than just being loosely contained in the pore space surrounding the three-dimensional scaffold. Moreover, 3D printing techniques, in particular 3D screen printing techniques, allow for a high degree of precision, reproducibility and an extremely high degree of resolution such that structures in the micrometre range can be reliably and reproducibly generated, i.e. printed. By such 3D printing techniques, in particular 3D screen printing techniques, the use of complicated manufacturing schemes involving production at different locations, transport of intermediate products between such different locations, and possibly further involving different production stages and multiple steps are avoided.

The thus generated three-dimensional biocompatible matrix is also structurally different from other matrices, such as bandages, dressings, patches, plasters, pouches, pads or gels, that may be commonly used in wound management. In particular, the three-dimensional scaffold of the three-dimensional biocompatible matrix according to the present invention (and thus the matrix itself) does not have a classical textile structure, in that it does not have a filamentous structure and/or is not a knitted fabric, and not a woven or non-woven. Moreover, the three-dimensional scaffold of the three-dimensional biocompatible matrix according to the present invention (and thus the matrix itself) also differs structurally from commercially available wound gels in that the three-dimensional scaffold is dimensionally stable and is not a spreadable semisolid or liquid, such as a cream, lotion, ointment, salve, paste, unguent, or balm. It is also not a spreadable or free-flowing gel, nor a gel soaked or imbibed into a gauze pad, nonwoven, woven, or other textile. Moreover, it is also not a gel that is structurally supported or held together by a textile or fibre mesh or other mesh.

Moreover, by an appropriate choice of said at least one component that is comprised by said three-dimensional biocompatible matrix, the qualities of such three-dimensional biocompatible matrix may be tailored to specific needs and/or requirements. For example, if it is necessary to alleviate pain caused by a wound, said at least one component may be a non-opioid analgesic. If, however, treatment and management of an inflammatory aspect of a wound is of primary importance, then the three-dimensional biocompatible matrix may comprise extracellular vesicles that contain appropriate cytokines, growth factors and/or mRNAs that might address inflammation. One advantage such extracellular vesicles is that they are easily incorporated into cells that form part of the affected tissue. If, for a lack of appropriate cells as an origin for such extracellular vesicles, no extracellular vesicles are available, then one may nevertheless consider including artificially constructed lipid vesicles in the three-dimensional biocompatible matrix, that have been loaded with desired ingredient(s) to bring about the desired effect. For example, such artificially constructed lipid vesicles may also be loaded with a non-opioid analgesic as defined herein.

In a preferred embodiment, the "polymeric agent", as used herein, is a gel-forming agent. The term "gel-forming agent", as used herein, is meant to refer an agent that is capable of forming a gel when exposed to a suitable liquid phase. The term "gel" as used herein, is meant to refer to a three-dimensional network of a solid, such three-dimensional network having pores or an interstitial pore space filled by a liquid phase. When such liquid phase is water or an aqueous solution, the resulting gel is also referred to as a "hydrogel". If the liquid is an organic solvent, the resulting gel is also referred to as an "organogel". Examples of suitable organic solvents are ethanol, acetone, isopropanol, glycerol, PEG-400, 3-methoxy-3-methyl-1-butanol (MBB), propylene carbonate, and vegetable oils. In preferred embodiments herein, a gel-forming agent is a hydrogel-forming agent, and the liquid phase that is used and comprised by the three-dimensional biocompatible matrix in such preferred embodiments is water or an aqueous solution. In such aqueous solution, additional components that may be comprised are buffer components, preservatives, chelating agents, humectants, dexpanthenol etc. When water or such aqueous solution is comprised by the three-dimensional biocompatible matrix, the three-dimensional scaffold is in a hydrogel-state or is a hydrogel.

The term "extracellular vesicles", as used herein, refers to particles that have been shed or released by biological cells. Typically, such extracellular vesicles have a lipid bilayer membrane and a size in the range of from 20 nm to 500 nm, preferably 20 nm to 300 nm, more preferably 20 nm to 200 nm, even more preferably 20 nm to 150nm, yet even more preferably 20 nm to 100 nm. In a particularly preferred embodiment, such extracellular vesicles have a lipid bilayer membrane and a size in the range of from 30 nm to 100 nm. In the phrase "extracellular vesicles derived from animal cells, preferably derived from mammalian cells, more preferably derived from human cells", the term "derived from animal cells, preferably derived from mammalian cells, more preferably derived from human cells ", is meant to refer to a scenario wherein such extracellular vesicles have been released from such animal cells, preferably derived from mammalian cells, more preferably derived from human cells. In a preferred embodiment, such extracellular resides have been released from human cells. For example, such extracellular vesicles may have been released from human platelets or stem cells. Depending on their respective cellular origin, the respective extracellular vesicles may differ in the composition of their respective membrane (and associated cell-surface markers) and/or their content. Typically, extracellular vesicles have a content and a surface that is reflective of the vesicles' cellular origin. They may contain proteins and/or fragments thereof, as well as nucleic acids, all originating from the vesicles' origin. In embodiments according to the present invention, the extracellular vesicles may contain one or several components selected from cytokines, growth factors, transcription factors, proteolytic enzymes, RNAs, in particular micro-RNA, and mRNA. Such components may have an anti-inflammatory or pro-inflammatory effect, for example they may be anti-inflammatory or pro-inflammatory cytokines. Proteolytic enzymes may contribute to the debridement of a wound. Methods for the production of extracellular vesicles are known to persons skilled in the art and have for example been described in Doyle et al., Cells, 2019, volume 8, 727, doi: 10.3390/cells8070727.

The term "artificially constructed lipid vesicles", as used herein, shall refer to vesicles having a lipid membrane, normally also a lipid bilayer membrane, and having been artificially synthesised instead of having been released from biological cells. Such artificially constructed lipid vesicles are also sometimes referred to as "liposomes". Such artificially constructed lipid vesicles have sizes in the range of from 20 nm to 150 nm, preferably 20 nm to 100 nm. Their outside shell Is made up of a lipid membrane, typically a lipid bilayer membrane, surrounding a core formed by a liquid phase. In accordance with embodiments of the present invention, such artificially constructed lipid vesicles may contain proteins and/or fragments thereof, as well as nucleic acids. In one embodiment, such artificially constructed lipid vesicles may contain one or several components selected from cytokines, growth factors, transcription factors, proteolytic enzymes, RNAs, in particular micro-RNA, and mRNA. In some embodiments, such artificially constructed lipid vesicles may also contain a non-opioid analgesic.

The term "biocompatible", as used herein in conjunction with said three-dimensional matrix is meant to refer, in the context of wound management, to the quality of such matrix of not eliciting any undesirable local or systemic effect in a recipient of such matrix, but generating a beneficial cellular response or tissue response, preferably an appropriate beneficial cellular or tissue response, more preferably the most appropriate beneficial cellular or tissue response, in such recipient of such matrix.

The term "resorbable", as used herein in the context of a three-dimensional scaffold according to the present invention is meant to refer to the quality of such scaffold to become resorbed by a living tissue to which said scaffold (and thus the matrix comprising such scaffold) has been applied. Resorption may for example occur by degradation and subsequent metabolisation of said scaffold.

Further aspects of the present invention are illustrated and exemplified by the following schemes, examples, tables and procedural descriptions which are given merely to illustrate, not to limit the present invention. The scope of protection for the present invention is merely limited by the appended claims.

In the following, reference is made to the examples, which are given to illustrate, not to limit the present invention.

### EXAMPLES

### Example 1:

Manufacturing of various inks (1.1 - 1.6) for three-dimensional (3D) screen printing of biocompatible matrix, and manufacturing of ink 1.7 for three-dimensional (3D) screen printing of backing layer

### 1.1

An amount of 12 g gelatin is solubilized in 200 ml PBS at 40°C under constant stirring of 200 rpm. After complete dissolution, 10 g of cellulose is added and stirred for 10 minutes. The stirring is increased to 400 rpm and 2 g alginate is added to the ink and stirred for 2 hours.

The ink is stored at 4°C till further use.

### 1.2

2 g collagen is added to 200 ml H2O and stirred for 1 h at 200 rpm at RT. Following complete dispersion or dissolution, 5 g cellulose and 0.5 g xanthan gum are added and stirred for 1h.

The ink is stored at 4°C till further use.

### 1.3

100 ml of 1.2% collagen dispersion is added to 100 ml H2O. The pH is controlled and adjusted to pH <7. Following complete dispersion, 2 g alginate is added and stirred for 2h.

The ink is stored at 4°C till further use.

### 1.4

2 g cellulose is added to 200 ml H2O and incubated overnight at 4°C. Next day, the soaked cellulose is stirred for 1h at RT. 10 ml Glycerol and 1 g PEG are added and stirred for 1h at 400 rpm. 1.5 g collagen is added and stirred for 2h at 400 rpm. The bioink is further mixed in a speedmixer for 10 minutes with increasing speed from 800 to 1500 rpm.

The ink is stored at 4°C till further use.

### 1.5

An amount of 2 g alginate is solubilized in 50 ml PBS or ddH₂O and mixed with 20 ml of 0.9% collagen suspension under constant stirring of 200 rpm. The obtained bioink is further supplemented with the non-opioid analgesic, and/or EVs. To this end, 2.5 g non-opioid analgesic and 2.5 g PVP are solubilized in 20 ml of CO₂ enriched water. This non-opioid analgesic/PVP suspension is then added to the bioink and shortly mixed. Furthermore, 10 ml of an EV/PBS suspension (extracellular vesicles in phosphate buffered saline) with an EV concentration of 2.5×10⁹/ml is added.

The ink is stored at 4°C till further use.

### 1.6

An amount of 2.5 g alginate and 5.5 g gelatin are solubilized in 100 ml PBS, ddH₂O, or CO2 enriched water under constant stirring of 400 rpm at 40°C. Furthermore, 5 g non-opioid analgesic and 5 g PVP are solubilized in the obtained bioink.

The ink is stored at 4°C till further use.

### 1.7

180g silicon component A is added and stirred with 20g silicon component B. 0,1- 0,5w% of a thixotropic thickner and 0,5-4w% curing time inhibitor is added and stirred. Finally, 1-4w% colour paste is added. The silicon paste is further mixed in a speedmixer for 10 minutes with increasing speed from 800-1500rpm.

### Example 2:

### Manufacture of three-dimensional biocompatible matrix

### A)

The ink composition for 3D printing 1.1. to 1.6 is used to produce a three-dimensional biocompatible matrix in a 3D screen printing unit. The ink from 1.1. to 1.6 is pre-heated or precooled to a temperature of 4-50°C, preferably 20°C. The screen printing squeegees are used with an angle of 50°-80°, preferably 65° and a hardness of 65-85 Shore for flooding, preferably 75 Shore, and 40-90 Shore for printing, preferably 55 Shore. The screen mesh is composed of polyamide, polyester, or steel with 8-400 threads/cm, preferably 48 threads/cm, and a thread thickness of 27-300 µm, preferably 55 µm. The screen EOM ("Emulsion over Mesh") can vary between 8-150 µm, preferably 20-25 µm. The printing process to build-up the geometry in z-axis is a continuous repetition of flooding, printing, and curing. The ink is flooded and printed through the screen mesh with the squeeguees at a temperature between 4-65°C, preferably 20-40°C. Humidity in the printing chamber during the printing process is adjusted to 20-100%, preferably 60%. The curing of the printed ink is executed at 0-180°C using IR, convection drying, UV, or a cooling system. Preferably, the bioinks from 1.1. to 1.6 are cured at temperatures between 4-40°C. Humidity in the curing chamber can be adjusted to 10-60%, preferably 40%. The printing process is repeated until the final size of the three-dimensional biocompatible matrix is achieved. The printing process can involve a combination and/or rotation of the inks 1.1 to 1.6. Furthermore, the printing process can involve several screens with different mesh compositions and geometries. When inks 1.5 or 1.6 are used, the active ingredient, i.e. the "at least one component selected from a non-opioid analgesic, extracellular vesicles derived from animal cells, preferably derived from mammalian cells, more preferably derived from human cells, and artificially constructed lipid vesicles" becomes incorporated into the three-dimensional biocompatible matrix by and during the printing process itself. If, however, inks 1.1 - 1.4 are used, such active ingredient, i.e. the "at least one component selected from..." has to be added separately to the already printed scaffold/matrix, as will be described in the following:

### B)

The ink composition for 3D printing 1.1. to 1.4 is used to produce a three-dimensional biocompatible matrix in a 3D screen printing unit. The ink from 1.1. to 1.4 is pre-heated or precooled to a temperature of 4-50°C, preferably 20°C. The screen printing squeegees are used with an angle of 50°-80°, preferably 65° and a hardness of 65-85 Shore for flooding, preferably 75 Shore, and 40-90 Shore for printing, preferably 55 Shore. The screen mesh is composed of polyamide, polyester, or steel with 8-400 threads/cm, preferably 48 threads/cm, and a thread thickness of 27-300 µm, preferably 55 µm. The screen EOM ("Emulsion over Mesh") can vary between 8-150 µm, preferably 20-25 µm. The printing process to build-up the geometry in z-axis is a continuous repetition of flooding, printing, and curing. The ink is flooded and printed through the screen mesh with the squeeguees at a temperature between 4-65°C, preferably 20-40°C. Humidity in the printing chamber during the printing process is adjusted to 20-100%, preferably 60%. The curing of the printed ink is executed at 0-180°C using IR, convection drying, UV, or a cooling system. Preferably, the bioinks from 1.1. to 1.4 are cured at temperatures between 4-40°C. Humidity in the curing chamber can be adjusted to 10-60%, preferably 40%. The printing process is repeated until the final size of the three-dimensional biocompatible matrix is achieved. The printing process can involve a combination and/or rotation of the inks 1.1 to 1.4. Furthermore, the printing process can involve several screens with different mesh compositions and geometries.

Subsequently, the desired active ingredient(s), i.e. the "at least one component selected from ...", e.g.non-opioid analgesic/EVs are introduced into the three-dimensional biocompatible scaffold/matrix by applying such "at least one component selected from ..." to or onto such scaffold/matrix. The step of applying such component can be performed by any suitable technology, including spraying, soaking, coating, imbibing, impregnating, applying and subsequently freeze-drying, nanosol technology, applying and subsequently exposing scaffold/matrix to negative pressure or vacuum, etc..

### Example 3:

### Manufacture of a backing layer and application thereof to three-dimensional (3D) biocompatible matrix

To maximize the stability of the 3D screen printed three-dimensional biocompatible matrices, crosslinking agents can be used during the printing process, or thereafter.

The silicon-based composition for 3D printing 1.7 of Example 1, above, is used to produce a three-dimensional backing layer. The paste from 1.7 is processed under room temperature conditions in the screen-printing unit. The screen-printing squeegees are used with an angle of 50°-80°, preferably 65° and a hardness of 65-85 Shore for flooding, preferably 75 Shore, and 40-90 Shore for printing, preferably 55 Shore. The screen mesh is composed of polyamide, polyester or steel with 8-400 threads/cm, preferably 48 threads/cm, and a thread thickness of 27-300 µm, preferably 55 µm. The screen EOM can vary between 8-150 µm, preferably 20-25 µm. The printing process to build-up the geometry in z-axis is a continuous repetition of flooding, printing and curing. The silicon paste is flooded and printed through the screen mesh with the squeeguees at room temperature. The curing of the printed silicon paste is executed at 120-180°C for 10-50 seconds, preferably 20-30 seconds using IR and/or convection drying. The printing process is repeated until the final size of the three-dimensional backing layer is achieved. The printing process can involve several screens with different mesh compositions and geometries to variate the backing layer. Such backing layer can subsequently be applied to any of the three-dimensional (3D) biocompatible matrices of Example 2, and can be affixed thereto by gluing, attaching, laminating etc., or by affixing it thereto using one or several additional adhesive layers.

One to several adhesive layers to finalize the product can potentially be applied between 1.1 to 1.6 and 1.7 by 3D screen printing. For this, a pressure sensitive adhesive paste is processed under room temperature conditions in the screen-printing unit as described before. Therefore 5-20 layers each with a thickness of 5-25 µm, preferably 10-20 µm of the pressure sensitive adhesive were printed as a separate carrier matrix.

The features of the present invention disclosed in the specification, the claims, and/or in the accompanying figures may, both separately and in any combination thereof, be material for realizing the invention in various forms thereof.

## Claims

1. A three-dimensional biocompatible matrix comprising
- a three-dimensional scaffold made of a polymeric agent, and
- at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm, said at least one component being incorporated in said scaffold.

2. The three-dimensional biocompatible matrix according to claim 1, wherein said scaffold is dimensionally stable and is not a spreadable semi solid or liquid, such as a cream, lotion, ointment, salve, paste, unguent, or balm.

3. The three-dimensional biocompatible matrix according to any of claims 1 - 2, wherein said scaffold has been produced by three-dimensional (3D) printing, preferably by three-dimensional (3D) screen printing,

4. The three-dimensional biocompatible matrix according to any of claims 1 - 3, wherein said scaffold is not a knitted fabric, not a woven or non-woven, and/or does not have a filamentous structure, wherein, preferably, said scaffold is resorbable.

5. The three-dimensional matrix according to any of claims 1 - 4, wherein said scaffold has a thickness in the range of from 100 µm to 20 mm, preferably from 100 µm to 15 mm, more preferably from 100 µm to 12 mm, even more preferably from 100 µm to 10 mm, wherein even more preferably said scaffold has a minimum thickness in the range of from 100 µm to 200 µm, allowing for the accommodation of a monolayer of cells, in particular of fibroblasts and/or keratinocytes.

6. The three-dimensional biocompatible matrix according to any of the foregoing claims, comprising
a)
- a scaffold made of a polymeric agent, and
- a non-opioid analgesic, incorporated in said scaffold or
b)
- a scaffold made of a polymeric agent, and
- extracellular vesicles derived from animal cells, preferably derived from mammalian cells, more preferably derived from human cells, incorporated in said scaffold; or
c)
- a scaffold made of a polymeric agent, and
- artificially constructed lipid vesicles having a size in the range of from 20 nm to 150 nm, incorporated in said scaffold; or
d)
- a scaffold made of a polymeric agent, and
- a non-opioid analgesic and extracellular vesicles derived from animal cells, preferably derived from mammalian cells, more preferably derived from human cells, both said non-opioid analgesic and said extracellular vesicles being incorporated in said scaffold; or
e)
- a scaffold made of a polymeric agent, and
- a non-opioid analgesic and artificially constructed lipid vesicles having a size in the range of from 20 nm to 150 nm, both said non-opioid analgesic and said artificially constructed lipid vesicles being incorporated in said scaffold; or
f)
- a scaffold made of a polymeric agent, and
- non-opioid analgesic and extracellular vesicles derived from animal cells, preferably derived from mammalian cells, more preferably derived from human cells and artificially constructed lipid vesicles having a size in the range of from 20 nm to 150 nm, each of them being incorporated in said scaffold.

7. The three-dimensional biocompatible matrix according to any of claims 1 - 6, wherein said polymeric agent is selected from the group comprising a) naturally occurring polymers selected from polysaccharides, e.g. agarose, chitin, chitosan, dextran, alginate, carrageenan, cellulose, starch, fucoidan, laminaran, glycosaminoglycans, copolymers of glycosaminoglycan and collagen; gums selected from xanthan gum, arabic gum, ghatti gum, guar gum, locust bean gum, tragacanth gum, karaya gum; and inulin; polypeptides, e.g. collagen, and hydrolysed forms of such polypeptides, e.g. gelatin; poly-amino acids, such as poly-lysine; polynucleotides; b) synthetic polymers, such as poly(alpha-hydroxy acids), such as poly lactic acid (PLA), poly glycolic acid (PGA), poly lactic-co-glycolic acid (PLGA), poly(alkyl)methacrylates, e.g. poly(methyl)methacrylates, polyvinylpyrrolidone (PVP), polyethylene glycol (PEG), polyurethanes, polycaprolactons, polyamides, polydioxanones, polyglycerol, and mixtures or combinations of such synthetic polymers b) with such naturally occurring polymers a); c) silicone-based polymers, such as polysiloxanes, e.g. polydimethylsiloxanes; and combinations or mixtures of any of the foregoing of a) - c).

8. The three-dimensional biocompatible matrix according to claim 7, wherein said polymeric agent is selected from naturally occurring polymers selected from polysaccharides, e.g. agarose, chitin, chitosan, dextran, alginate, carrageenan, cellulose, starch, fucoidan, laminaran, glycosaminoglycans, copolymers of glycosaminoglycan and collagen; gums selected from xanthan gum, arabic gum, ghatti gum, guar gum, locust bean gum, tragacanth gum, karaya gum; and inulin; polypeptides, e.g. collagen, and hydrolysed forms of such polypeptides, e.g. gelatin; poly-amino acids, such as poly-lysine; polynucleotides; wherein, preferably said polymeric agent is selected from alginate, gelatin, cellulose, collagen, chitosan, and mixtures of any of the foregoing.

9. The three-dimensional biocompatible matrix according to any of the foregoing claims, wherein said non-opioid analgesic is selected from amino ester analgesics, amino amide analgesics, fomocains, and respective carbonic acid adducts of said amino ester analgesics or of said amino amide analgesics or of said fomocains, wherein preferably, said amino ester analgesics include procaine, chloroprocaine, oxybuprocaine, benzocaine, tetracaine, and proxymetacaine; and wherein, preferably, said amino amide analgesics include lidocaine, mepivacaine, prilocaine, articaine, bupivacaine, ropivacaine, cinchocaine, and etidocaine; and wherein, preferably, said fomocains include fomocaine and C-alkylmorpholine derivatives thereof.

10. The three-dimensional biocompatible matrix according to any of the foregoing claims wherein said non-opioid analgesic is an amino ester analgesic or a carbonic acid adduct thereof, preferably selected from procaine, chloroprocaine, oxybuprocaine, benzocaine, tetracaine, and proxymetacaine, and their respective carbonic acid adducts.

11. The three-dimensional biocompatible matrix according to any of claims 1 - 9, wherein said non-opioid analgesic is an amino amide analgesic or a carbonic acid adduct thereof, preferably selected from lidocaine, mepivacaine, prilocaine, articaine, bupivacaine, ropivacaine, cinchocaine, and etidocaine, and their respective carbonic acid adducts.

12. The three-dimensional biocompatible matrix according to any of claims 1 - 8, wherein said non-opioid analgesic is selected from acetylsalicylic acid, ibuprofen, diclofenac, naproxen, indomethacin, paracetamol, metamizol, phenazone, propyphenazone, parecoxib, celecoxib, etoricoxib, ketamine, capsaicin, ziconotide, cannabinoids, and flupirtine.

13. The three-dimensional biocompatible matrix according to any of the foregoing claims, wherein said extracellular vesicles are derived from animal platelets or stem cells, preferably mammalian platelets or stem cells, more preferably human platelets or stem cells, and contain at least one component selected from cytokines, growth factors, transcription factors, RNAs, in particular micro RNA, and mRNA; wherein, preferably, said stem cells are selected from mesenchymal stem cells and induced stem cells, wherein, more preferably, said mesenchymal stem cells are derived from bone marrow, umbilical cord blood, adipose tissue, or amniotic fluid; and wherein, preferably, said artificially constructed lipid vesicles contain at least one component selected from cytokines, growth factors, transcription factors, RNAs, in particular micro RNA, mRNA, and a non-opioid analgesic, as defined in any of claims 9 - 12.

14. The three-dimensional biocompatible matrix according to any of the foregoing claims wherein a) said extracellular vesicles are derived from animal platelets, preferably mammalian platelets, more preferably human platelets, and are positive for at least one of cellular markers selected from CD9, CD41a, CD41b, CD42b, CD61, CD62P, CD63 and syntenin, and/or wherein said extracellular vesicles are negative for at least one of cellular markers selected from CD81, CD3, CD4, CD19, CD20, CD2, CD8, CD11a and CD25; or wherein b) said extracellular vesicles are derived from stem cells and are positive for CD81, CD9, CD63, Tsg101, and HSP70, and they are positive for one, several or all of CD105, CD90, CD73 and CD44, and/or wherein said extracellular vesicles are negative for one, several or all of CD45, CD34, CD31, CD19, CD79α, CD14, CD11b, and HLA-DR.

15. A composition comprising a three-dimensional biocompatible matrix according to any of the foregoing claims.

16. The composition according to claim 15, manufactured as a patch, dressing, pad or pouch.

17. The composition according to any of claims 15 - 16, which has one of the following two structures A or B:
**structure A:**
wherein said three-dimensional biocompatible matrix is arranged in a first layer, and wherein said composition comprises an additional layer attached to said first layer, which additional layer is either a carrier layer providing mechanical support for said biocompatible matrix, or is a semipermeable backing layer preventing diffusion of said at least one component from one side of said biocompatible matrix, or is a solvent-impermeable backing layer, in particular a water-impermeable backing layer, sealing said biocompatible matrix on one side and preventing diffusion of said at least one component from said biocompatible matrix on such side, or wherein said additional layer is both a carrier layer and a semipermeable or solvent-impermeable backing layer;
wherein said first layer has a thickness in the range of from 100 µm to 20 mm, preferably from 100 µm to 15 mm, more preferably from 100 µm to 12 mm, even more preferably from 100 µm to 10 mm; and wherein said additional layer has a thickness in the range of from 50 µm to 2mm, preferably 100 µm to 2mm, more preferably from 100 µm to 1mm, and even more preferably from 100 µm to 500 µm; or
**structure B:**
wherein said three-dimensional biocompatible matrix is arranged as the only layer within said composition, and there is no additional layer attached to said layer of said three-dimensional biocompatible matrix; and wherein said only layer has a thickness in the range of from 500 µm to 3 mm, preferably in the range of from 500 µm to 2 mm, more preferably in the range of from 1 mm to 2 mm;
wherein, optionally, said composition of structure A and said composition of structure B may be attached to a releasable carrier sheet for transport and/or storage of said composition, and is not attached to a releasable carrier sheet and does not comprise such releasable carrier sheet, when said composition is in use, e.g. in treating and/or managing a wound.

18. The composition according to claim 17, wherein said composition of structures A and B is attached to a releasable carrier sheet for transport and/or storage of said composition, and is not attached to a releasable carrier sheet and does not comprise such releasable carrier sheet, when said composition is in use, e.g. in treating and/or managing a wound.

19. The three-dimensional biocompatible matrix according to any of claims 1 - 14 or the composition according to any of claims 15 - 18, for use in a method of treating and/or managing a wound.

20. The three-dimensional biocompatible matrix or the composition for use according to claim 19, wherein said wound is an acute wound or a chronic wound, wherein, preferably, said acute wound is a burn, a skin lesion, a skin injury, a surgical wound, a cut or a stab wound, and wherein, preferably, said chronic wound is a decubitus ulcer or a diabetic ulcer; wherein, more preferably, said burn is selected from thermal burns, radiation burns, chemical burns, burns cause by exposure to electrical currents, and friction burns, wherein, even more preferably, said burn is a first degree, second degree or third degree burn, even more preferably a second degree or third degree burn.

21. The three-dimensional biocompatible matrix or composition for use according to any of claims 19 and 20, wherein said method of treating and/or managing a wound comprises administering said three-dimensional biocompatible matrix or said composition to said wound and allowing it to remain in contact with such wound for a defined period of time.

22. The three-dimensional biocompatible matrix or composition for use according to any of claims 19 - 21, wherein said method is for alleviating pain and/or for reducing inflammation of said wound.

23. The three-dimensional biocompatible matrix or composition for use according to any of claims 19 - 22, wherein said method is for increasing the speed at which wound healing is achieved in comparison to a wound that is not treated by said method, and/or wherein said method is for delivery of an analgesic to said wound, and/or wherein said method is for reconstruction of skin in and/or around said wound.

24. A method for producing a three-dimensional biocompatible matrix, as defined in any of claims 1 - 14, said method comprising
Either:
a) Providing a polymeric agent and 3D-printing, preferably 3D-screen-printing, such agent into a three-dimensional scaffold;
b) Applying at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm, to said three-dimensional scaffold and allowing said at least one component to be incorporated in said scaffold, thereby producing said three-dimensional biocompatible matrix;
said polymeric agent and said at least one component being as defined in any of claims 1 - 14;
Or:
a^{∗}) Providing a polymeric agent and at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm, and mixing said polymeric agent and said at least one component, and thereafter
b^{∗}) 3D-printing, preferably 3D-screen-printing, the resultant mixture from step a^{∗}) into a three-dimensional scaffold, such that said at least one component is incorporated in said scaffold, thereby producing said three-dimensional biocompatible matrix;
said polymeric agent and said at least one component being as defined in any of claims 1 - 14.

25. An ink composition for three-dimensional (3D) printing, in particular three-dimensional (3D) screen printing, said ink composition comprising:
i. a polymeric agent selected from the group comprising a) naturally occurring polymers selected from polysaccharides, e.g. agarose, chitin, chitosan, dextran, alginate, carrageenan, cellulose, starch, fucoidan, laminaran, glycosaminoglycans, copolymers of glycosaminoglycan and collagen; gums selected from xanthan gum, arabic gum, ghatti gum, guar gum, locust bean gum, tragacanth gum, karaya gum; and inulin; polypeptides, e.g. collagen, and hydrolysed forms of such polypeptides, e.g. gelatin; poly-amino acids, such as poly-lysine; polynucleotides; b) synthetic polymers, such as poly(alpha-hydroxy acids), such as poly lactic acid (PLA), poly glycolic acid (PGA), poly lactic-co-glycolic acid (PLGA), poly(alkyl)methacrylates, e.g. poly(methyl)methacrylates, polyvinylpyrrolidone (PVP), polyethylene glycol (PEG), polyurethanes, polycaprolactons, polyamides, and mixtures or combinations of such synthetic polymers b) with such naturally occurring polymers a); c) silicone-based polymers, such as polysiloxanes, e.g. polydimethylsiloxanes; and combinations or mixtures of any of the foregoing of a) - c);
ii. a solvent for i, wherein preferably said solvent is selected from water, aqueous solutions, alcohol, in particular methanol, ethanol, n-propanol, or isopropanol, alcoholic solutions, tetrahydrofuran, acetone, and ethyl acetate;
iii. at least one component selected from a non-opioid analgesic, extracellular vesicles derived from animal cells, preferably derived from mammalian cells, more preferably derived from human cells, and artificially constructed lipid vesicles having a size in the range of from 20 nm to 150 nm; wherein said non-opioid analgesic, extracellular vesicles derived from animal cells, preferably derived from mammalian cells, more preferably derived from human cells, and artificially constructed lipid vesicles are as defined in any of claims 9 - 14;
iv. optionally, one or several further components selected from fillers, cross-linkers, binders, surfactants, additives, diluents, thickening agents, colours, dyes, stabilizing agents, buffering agents, humectants, emulsifying agents, dispersing agents, and preserving agents.
